# EUROPEAN PATENT SPECIFICATION

(11) **EP 2 486 856 B1**
(45) Date of publication and mention of the grant of the patent: **09.07.2014**
(21) Application number: 12154469.6
(22) Date of filing: 08.02.2012
(51) Int. Cl.: A61B 17/04

(54) **Bone anchor for scapholunate construct**
Knochenanker für skapholunäres Rekonstruktion
Ancre pour os pour la reconstruction scapholunaire

(30) Priority: 09.02.2011 US 201161441146 P
(43) Date of publication of application: 15.08.2012
(73) Proprietor: Arthrex, Inc., Naples, FL 34108-1945 (US)
(72) Inventor: Choinski, Ronald J., Fort Myers Beach, Florida 33931 (US); Gallen, Karen L., Naples, Florida 34109 (US); Moore, Jesse G., Naples, Florida 34119 (US); Lee, Steve K., New York, New York 10003 (US); Yao, Jeffrey, Redwood City, California 94063 (US); Zlotolow, Dan A., Glenside, Pennsylvania 19038 (US)
(74) Representative: Benedum, Ulrich Max

(56) References cited:
- EP-A2- 1 491 162
- WO-A1-2009/055800
- US-A1- 2005 075 668
- US-A1- 2010 004 683

## Description

### FIELD OF THE INVENTION

The present invention relates to the field of surgery and, in particular, to devices for reattachment of interosseous ligaments such as the scapholunate ligament.

### BACKGROUND OF THE INVENTION

The wrist is a complex joint which relies on ligaments to provide a great degree of mobility for positioning the hand for function. The areas of the wrist most often affected by ligament injury are the joints of the first row of bones beyond the wrist joint, called the proximal carpal row, the particular joints affected being those between the scaphoid and lunate bones (scapholunate joint) and between the lunate and triquetral bones (lunotriquetral joint).

Scapholunate ligament injury allows the two bones to move apart, leaving a gap typically seen on an x-ray. The width of the gap is variable, but the most important effect of this injury is that the scaphoid is no longer properly controlled and falls into a flexed position (i.e., is bent forward). Smooth movement is no longer possible and the patient experiences clicking, pain and weakness. The instability can be progressive, with worsening symptoms and ultimately arthritic changes (known as scapholunate advanced collapse).

Various techniques for surgical repair of the scapholunate ligament are known in the art. These techniques typically involve the use of nearby tendon tissue to reconstruct ligaments between the two bones. This is supported with a temporary wire between the two bones to hold them together. The ligament repair takes about 7-8 weeks to heal, and the wire is then removed. It is usual that some movement (flexion and/or extension) is lost through operation, as joining of the two little, oddly-shaped bones (scaphoid and lunate) is difficult to achieve. The existing techniques also do not enable connecting biological graft directly to the fixation devices (i.e, to multiple anchors). Fusion of the scaphoid and lunate (an alternate to the wire repair technique) is also not indicated as it causes arthritis.

Different anchors for attachment of soft tissue to bones can be found in the prior art. US 2005/0075668 discloses an anchor according to the introductory portion of independent claim 1 and US 2010/0004683 discloses an anchor comprising: a body with a central axis, a proximal end, a distal end, an internal bore provided at the proximal end; two opposing lateral sides, and other two opposing lateral sides that are adjacent the two opposing flat lateral sides and are provided with external non-flat surfaces, and an eyelet located at the distal end of the body, which is about half the length of the body.

### SUMMARY OF THE INVENTION

The present invention provides devices for reattachment of ligaments as an alternative to fusion. The devices may be applied to any interosseous ligament repairs, including scapholunate repairs, syndesmotic injuries, or hallux valgus repairs, among others. Particular applicability is to scapholunate ligament repairs, as the scapholunate joint is exceptionally small and requires specialized devices and techniques.

The present invention provides a scapholunate dissociation/instability construct including an anchor having a proximal end and a distal end, and a threaded internal bore which may communicate with an eyelet at the distal end. The anchor is provided with two opposing lateral, parallel sides that are smooth and non-threaded, and two adjacent opposing, parallel sides that are provided with a plurality of partial ribs or partial threads.

An exemplary method of biologic scapholunate interosseous ligament reconstruction using the devices of the present invention comprises *inter alia* the steps of: (i) inserting positioning pins into the scaphoid and the lunate; (ii) reducing the scapholunate dissociation by maneuvering the two pins; (iii) drilling a hole through the central axis of the scaphoid and the lunate; (iv) providing the anchor construct of the present invention with tendon (biologic material) attached thereto, and placing the anchor construct with the attached tendon into the ulnar portion of the lunate; (v) optionally, pulling on the tails of the tendon tight while reduction of the scapholunate interval is maintained; and (vi) inserting a fixation device (such as a tenodesis screw) to fix the tendon strands to the scaphoid.

### BRIEF DESCRIPTION OF THE DRAWINGS

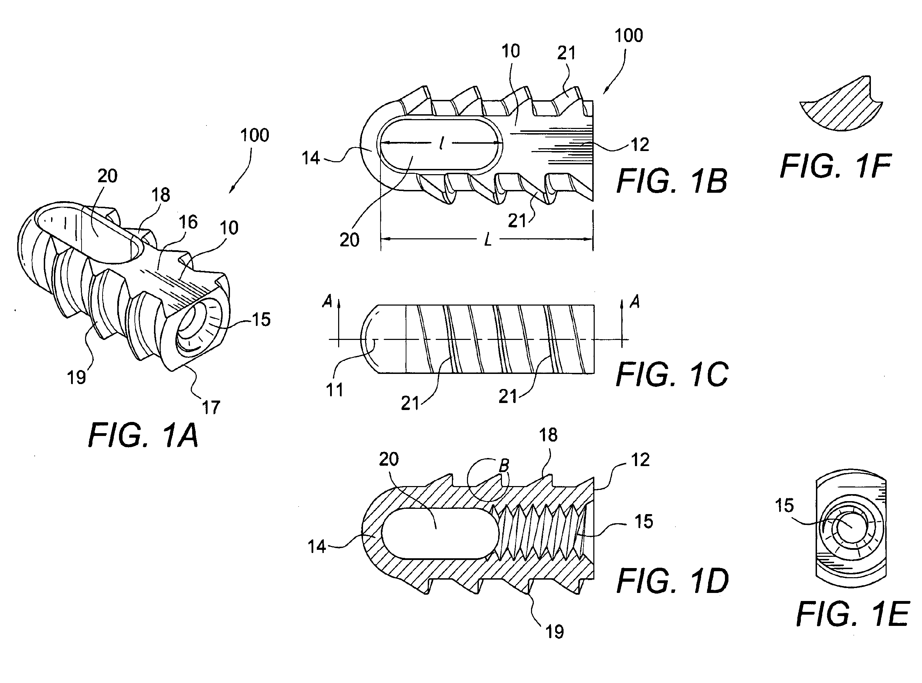

FIG. 1A illustrates a perspective view of a first embodiment of a 3.5mm anchor construct employed in a method of scapholunate interosseous ligament reconstruction of the present invention.

FIG. 1B illustrates a top view of the anchor construct of FIG. 1A.

FIG. 1C illustrates a plan view of the anchor construct of FIG. 1A.

FIX. 1D illustrates a cross-sectional view of the anchor construct of FIG. 1A taken along line A-A of FIG. 1C.

FIG. 1E illustrates a right side view of the anchor construct of FIG. 1A.

FIG. 1F illustrates an enlarged view of detail B of the anchor construct of FIG. 1D.

FIG. 2A illustrates a perspective view of a second embodiment of a 3.5mm anchor construct employed in a method of scapholunate interosseous ligament reconstruction of the present invention.

FIG. 2B illustrates a top view of the anchor construct of FIG. 2A.

FIG. 2C illustrates a plan view of the anchor construct of FIG. 2A.

FIG. 2D illustrates a cross-sectional view of the anchor construct of FIG. 2A taken along line A-A of FIG. 2C.

FIG. 2E illustrates a right side view of the anchor construct of FIG. 2A.

FIG. 3A illustrates a perspective view of a third embodiment of a 3.5mm anchor construct employed in a method of scapholunate interosseous ligament reconstruction of the present invention.

FIG. 3B illustrates a top view of the anchor construct of FIG, 3A.

FIG. 3C illustrates a plan view of the anchor construct of FIG. 3A.

FIG. 3D illustrates a cross-sectional view of the anchor construct of FIG. 3A taken along line A-A of FIG. 3C.

FIG. 3E illustrates a right side view of the anchor construct of FIG. 3A.

FIG. 3F illustrates an enlarged view of detail B of the anchor construct of FIG. 3D.

FIG. 4 illustrates a schematic view of a human wrist (identifying the scaphoid and the lunate bones).

FIGS. 5-15 illustrate subsequent steps of a first method of scapholunate interosseous ligament reconstruction.

FIG. 15(a) is a schematic illustration of the final scapholunate interosseous ligament repair of FIG. 15.

FIGS. 16-22 illustrate subsequent steps of a second method of scapholunate interosseous ligament reconstruction (by an alternate graft docking with a suture loop).

### DETAILED DESCRIPTION OF THE INVENTION

The present invention provides novel reconstruction systems for connecting biological material directly to multiple anchor(s) in interosseous ligament reconstructions such as scapholunate interosseous ligament repair.

The reconstruction system of the present invention comprises a fixation device in the form of a small anchor and biological or synthetic tissue material (for example, tendon, ligament or graft) attached to the anchor (for example, looped through an eyelet of the anchor). The anchor with the attached tissue (attached biological or synthetic material) is placed into a socket, opening or tunnel of a first bone and the loose tissue strands (tails) are fixated into the second bone (which is adjacent the first bone) with a fixation device such as a tenodesis screw, for example. In an exemplary embodiment only, the reconstruction system of the present invention comprises a fixation device in the form of a small 3.5mm anchor and a tendon graft attached to the 3.5mm anchor (for example, looped through an eyelet of the anchor). The 3.5mm anchor with the attached tendon is placed into the ulnar portion of the lunate and the loose tendon strands (tendon tails) are fixated into the scaphoid with a fixation device such as a tenodesis screw, for example.

The tissue (biological or synthetic material such as tendon graft or ligament) may be threaded through the eyelet of the anchor before it is deployed into the first bone (i.e., the attached tissue and the anchor are simultaneously deployed into the first bone). Alternatively, the tissue may be "drawn" into the bone socket or tunnel of the first bone by pulling the suture tails through the anchor eyelet (i.e., the tissue is deployed/drawn into the first bone after deployment of the anchor into the first bone).

Referring now to the drawings, where like elements are designated by like reference numerals, FIGS. 1A-3F illustrate exemplary embodiments of anchor 100, 200, 300 of the present invention. In the embodiments below, anchor 100, 200, 300 is a specific 3.5mm scapholunate anchor employed for scapholunate interosseous ligament reconstruction. Although the embodiments below will be described with reference to a particular application of anchor 100, 200, 300 (i.e., with reference to a scapholunate ligament repair) and although reference to anchor 100, 200, 300 will be made to as 3.5mm scapholunate anchor 100, 200, 300, the invention is not limited to these embodiments, and encompasses anchors with application to any other interosseous ligament reconstructions, for example, syndesmotic injuries and hallux valgus repairs, among many others.

As shown in FIGS. 1A-1F, the 3.5mm scapholunate threaded anchor 100 has the general shape of a short anchor comprising a body 10 with a proximal end 12 and a distal end 14. An inner threaded through bore 15 is provided within the body 10, extending from the most proximal end of the body and along longitudinal (central) axis 11 of the anchor. Body 10 has a generally rectangular configuration and a top surface 16 and a bottom surface 17 that are smooth, and adjacent sides 18, 19 that are provided with a plurality of partial threads or ribs 21.

Top and bottom side surfaces 16, 17 are opposed side surfaces of body 10 that extend in a direction about parallel to the longitudinal (central) axis 11 of the body 10, Top and bottom side surfaces 16, 17 are provided with smooth, non-threaded and non-ribbed outer (external) surfaces. Adjacent opposing lateral sides 18, 19 are opposed side surfaces of body 10 that are provided with non-flat surfaces, for example, with threaded and/or ribbed outer (external) surfaces. In this manner, when anchor construct 100 is inserted into a tunnel or socket in bone (for example, into the lunate), tissue strands attached to the anchor construct 100 will extend along the flat, smooth external surfaces 16, 17, while the threaded and/or ribbed outer (external) sides 18, 19 engage the walls of the bone tunnel or socket, to ensure secure fixation of the anchor construct 100 within the bone tunnel or socket.

In the specific embodiment illustrated in FIGS. 1A-1F, bore 15 is in direct communication with eyelet 20 formed within the body 15 and at the distal end 14. The eyelet 20 occupies about half the volume of the body 15 to allow a tendon graft to attach thereto. As shown in FIG. 1B, for example, the eyelet has a length "1" which is about half the length "L" of the anchor body 10. In an exemplary embodiment only, length L is about 3.5mm.

In use, a tendon graft (or any biologic or synthetic material for ligament reconstruction) is passed through the eyelet 20 and looped over the smooth flat external surfaces/sides 16, 17 of body 10 for further manipulation and fixation into sockets/tunnels formed within two bones such as the scaphoid and the lunate bones, as detailed below. Anchor 100 may be cannulated (to allow insertion over a K-wire, for example), or may be preloaded with a suture strand and a needle (for example, with a 2-0 Fiberwire), or may be provided with a continuous suture loop attached to the anchor (i.e., with a continuous, uninterrupted suture loop attached to eyelet 20 of the anchor 100).

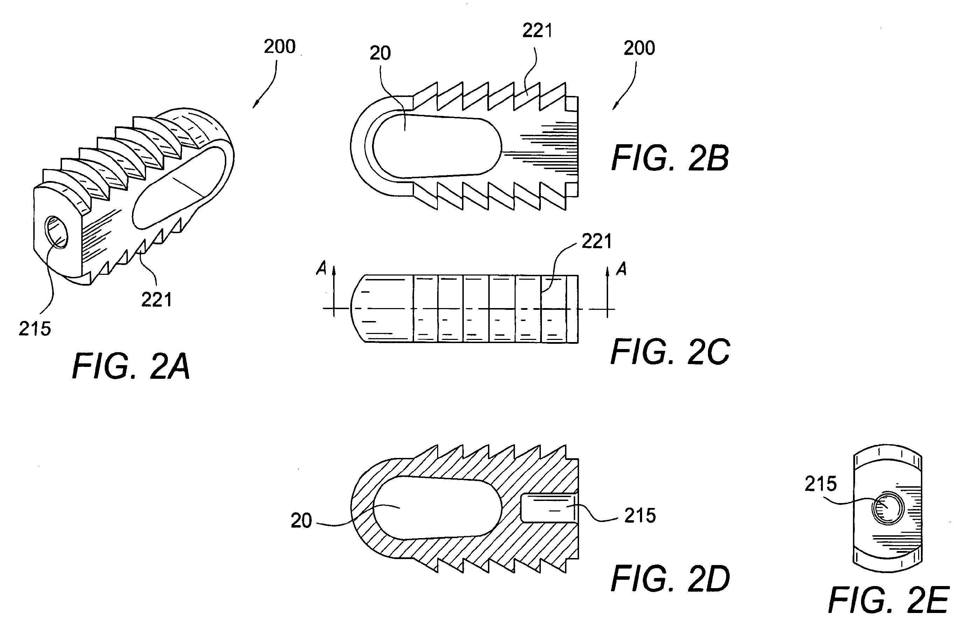

The 3.5mm scapholunate push-in anchor 200 of FIGS. 2A-2E is similar to the anchor 100 of FIGS. 1A-1F but differs in that bore 215 is not threaded and does not communicates with eyelet 20. Anchor 200 is also provided with a plurality of barbs 221 (having a conical configuration) *in lieu* of the partial threads 21.

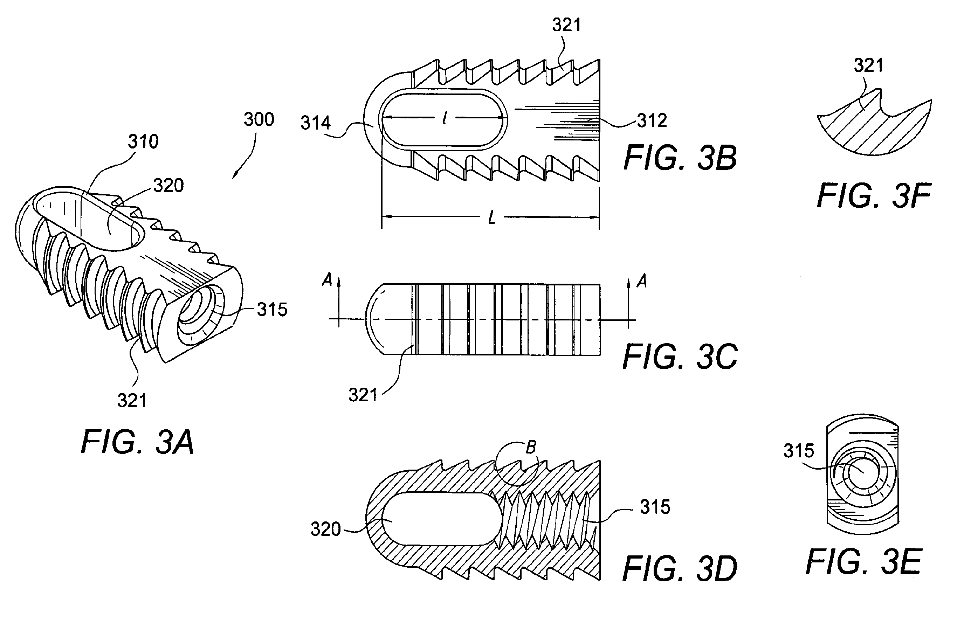

FIGS. 3A-3F illustrate yet another embodiment of a 3.5mm scapholunate anchor 300 of the present invention, which is a combination of the first and second embodiments detailed above, i.e., it is a push-in anchor with a threaded drive bore 315. As in the first embodiment, threaded drive bore 315 is in direct communication with eyelet 320 formed within the body 315 and at the distal end 314. The eyelet 320 occupies about half the volume of the body 315 to allow a tendon graft (or any biologic or synthetic material for ligament reconstruction) to attach thereto. Anchor 300 is also provided with a plurality of barbs 321 having a conical configuration to allow body 315 to be secured within a bone hole by simply pushing in the anchor body.

A method of biologic interosseous ligament reconstruction with anchor 100, 200, 300 of the present invention comprises *inter alia* the steps of: (i) forming (by drilling, for example) a hole or tunnel through the central axis of two adjacent first and second bones; (ii) providing the anchor construct 100, 200, 300 of the present invention with tissue (for example, biologic material such as tendon, graft or ligament, and/or synthetic material) attached thereto (by looping and folding the tissue over the eyelet, and extending the tissue strands along the flat side surfaces of the anchor, for example), and placing the anchor construct with the attached tissue into the first bone; (iii) optionally, pulling on the tails of the tissue (tendon) tight while reduction of the interval between the two bones is maintained; and (iv) inserting a fixation device (such as a tenodesis screw) to fix the tissue (tendon) strands to the second bone.

An exemplary method of biologic scapholunate interosseous ligament reconstruction using the device of the present invention comprises *inter alia* the steps of: (i) inserting positioning pins into the scaphoid and the lunate; (ii) reducing the scapholunate dissociation by maneuvering the two pins; (iii) drilling a hole through the central axis of the scaphoid and the lunate; (iv) providing the anchor construct of the present invention with tendon (biologic material) attached thereto, and placing the anchor construct with the attached tendon into the ulnar portion of the lunate; (v) optionally, pulling on the tails of the tendon tight while reduction of the scapholunate interval is maintained; and (vi) inserting a fixation device (such as a tenodesis screw) to fix the tendon strands to the scaphoid.

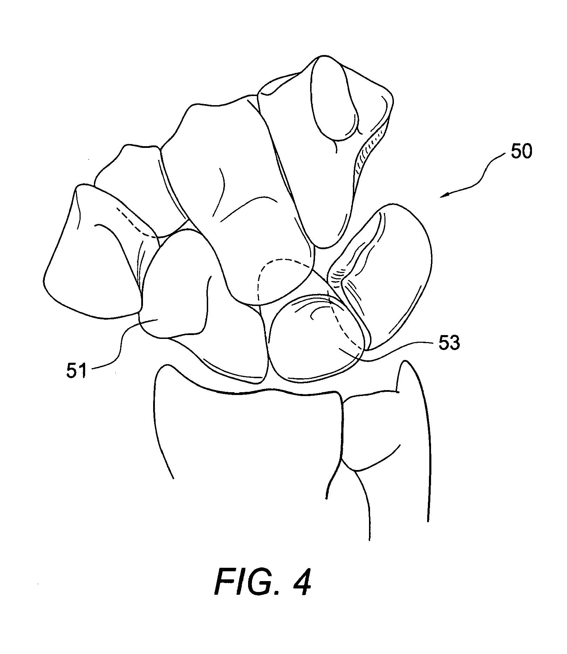

FIG. 4 illustrates a schematic representation of human wrist 50 with scaphoid 51 and lunate 53 undergoing an exemplary method of scapholunate interosseous ligament reconstruction and employing the 3.5mm scapholunate anchor 100, 200, 300 of FIGS. 1A-3F.

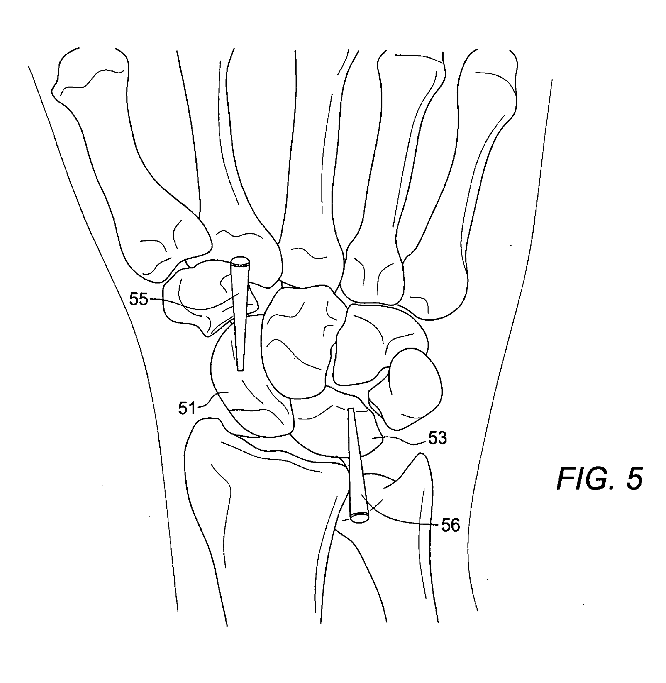

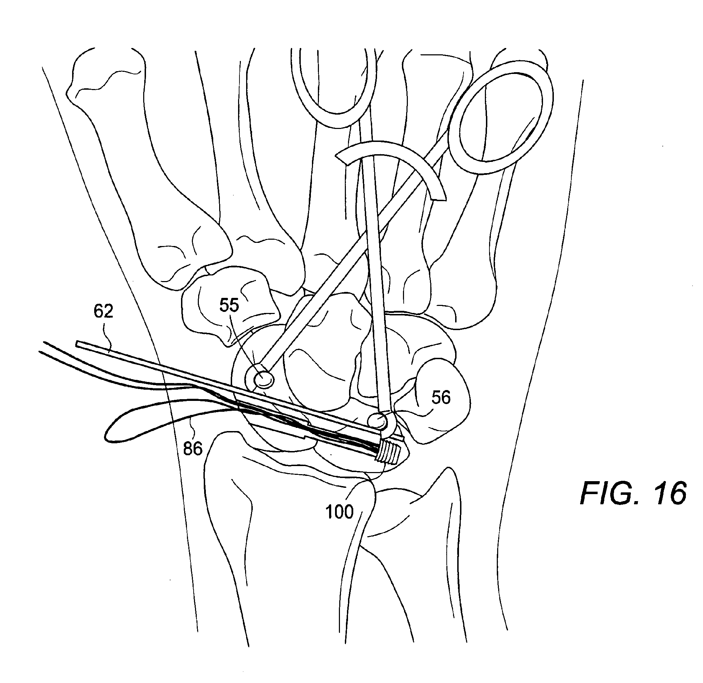

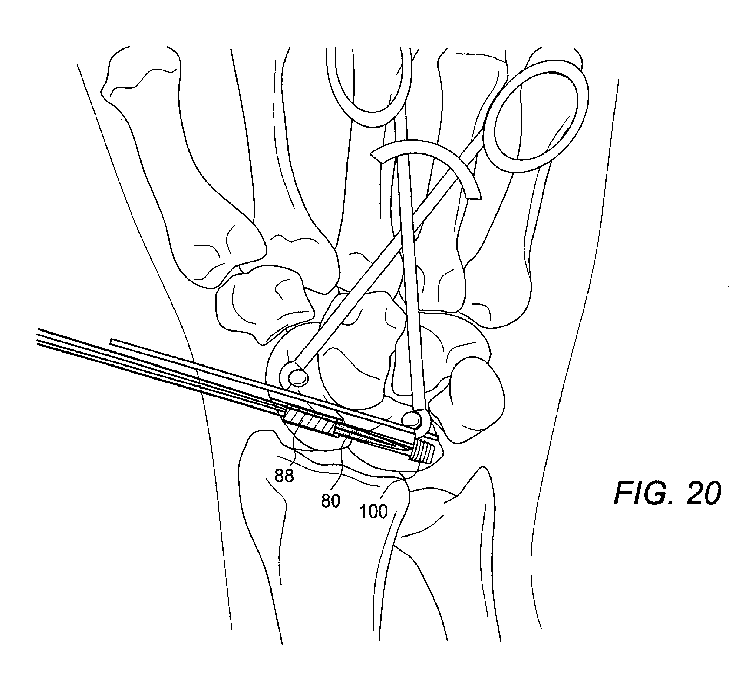

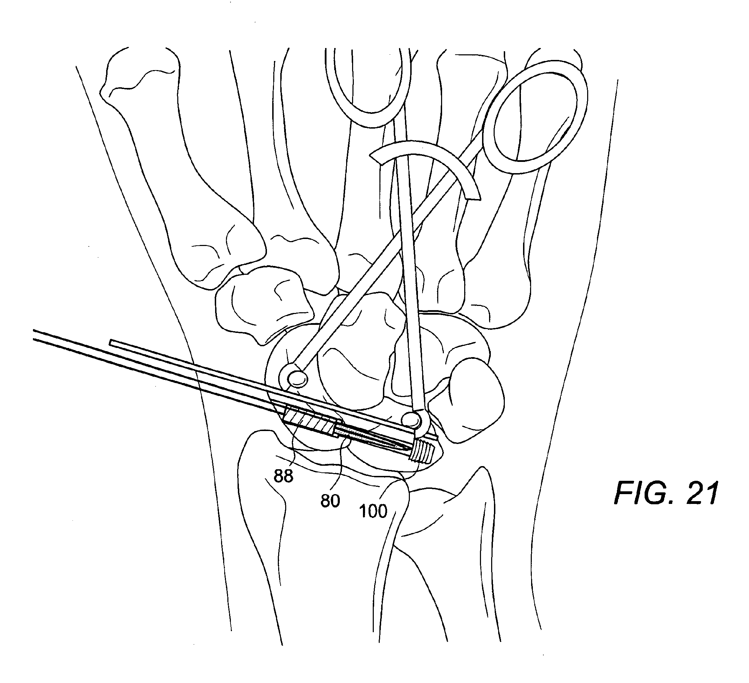

FIGS. 5-15 illustrate subsequent steps of a first method of scapholunate interosseous ligament reconstruction, while FIGS. 16-22 illustrate subsequent steps of a second method of scapholunate interosseous ligament reconstruction (with an alternate graft docking with a suture loop). Both exemplary methods are conducted with the exemplary 3.5mm scapholunate threaded anchor 100 of FIGS. 1A-1F.

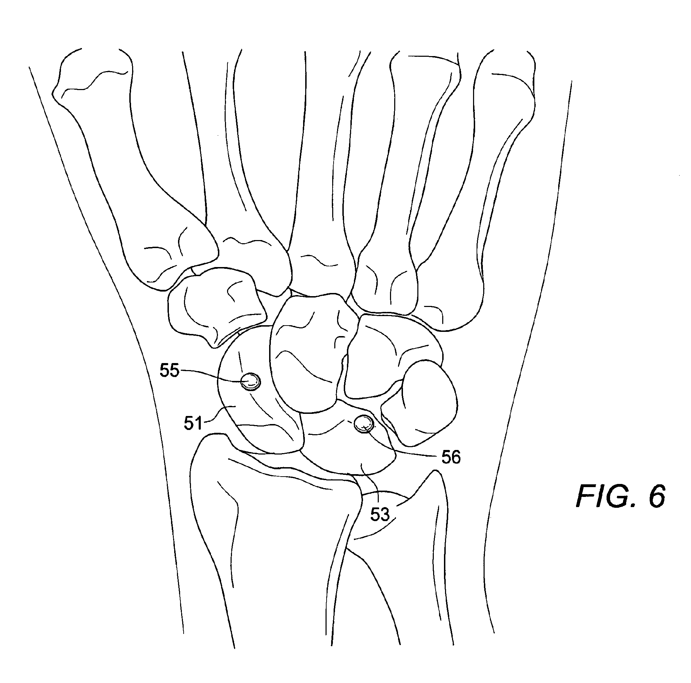

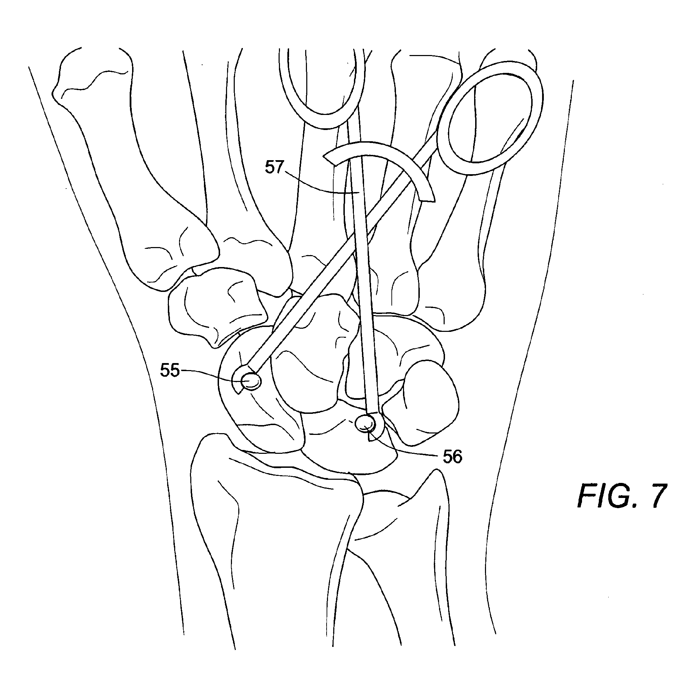

Reference is now made to FIGS. 5-15. Positioning pins 55, 56 are inserted into the scaphoid 51 and the lunate 53 (FIG. 5). The pins 55, 56 are fully inserted into the scaphoid 51 and lunate 53 to reposition the two bones (FIG. 6). As shown in FIG. 7, the scapholunate dissociation is reduced by maneuvering the two pins 55, 56 with, for example, a reduction clamp 57 having parallel channels that maintain angular reduction and compress the scapholunate gap.

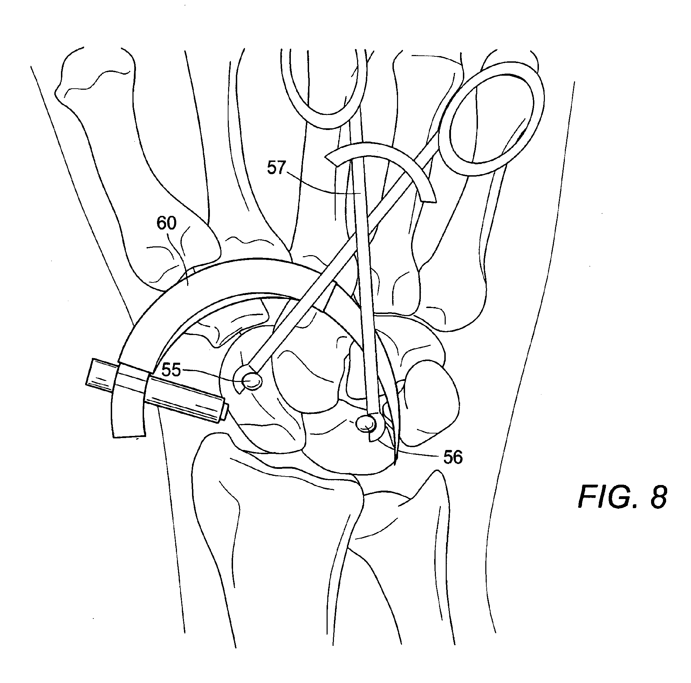

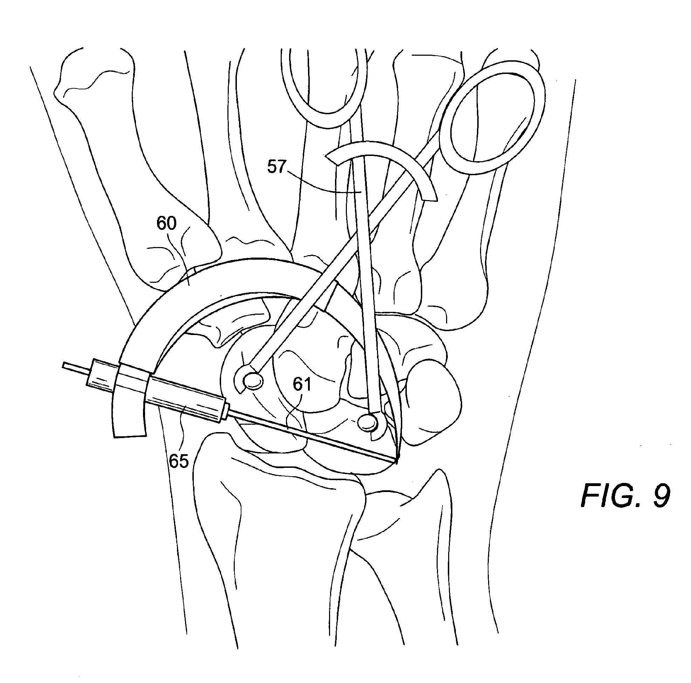

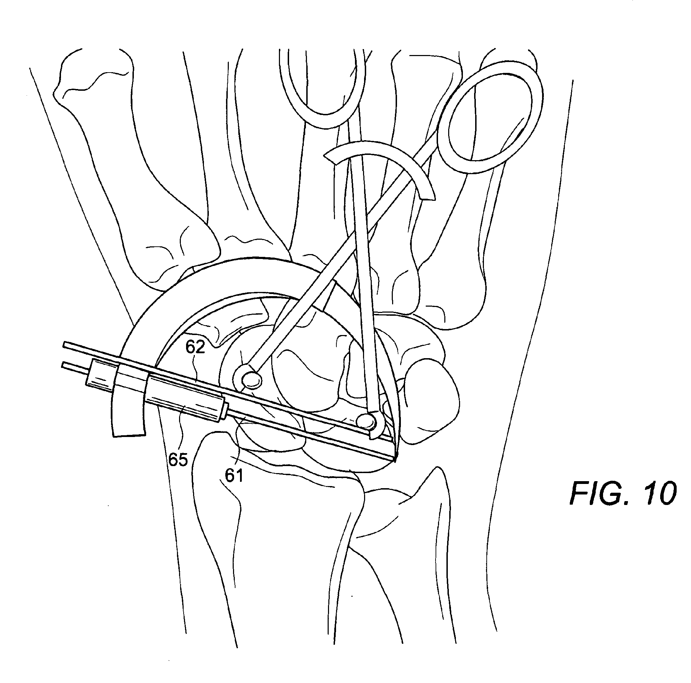

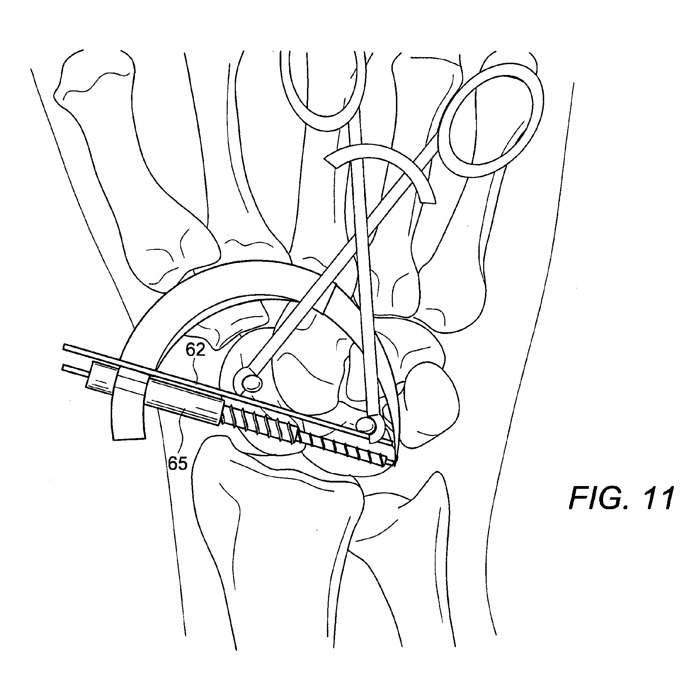

FIG. 8 illustrates placement of drill guide 60 once the gap between the scaphoid 51 and the lunate 53 has been reduced. A K-wire 61 is inserted through the central axis of scaphoid 51 and the lunate 53, as shown in FIG. 9. A second K-wire 62 may be placed through the scaphoid 51 and the lunate 53, and adjacent the first K-wire 61, to prevent rotational movements (and as shown in FIG. 10). The centrally-placed first K-wire 61 is then over drilled with an appropriate stepped drill 65. The stepped drill underdrills the distal end of the hole where the anchor 100 is inserted and overdrills the proximal end of the hole to create room for the graft (FIG. 11).

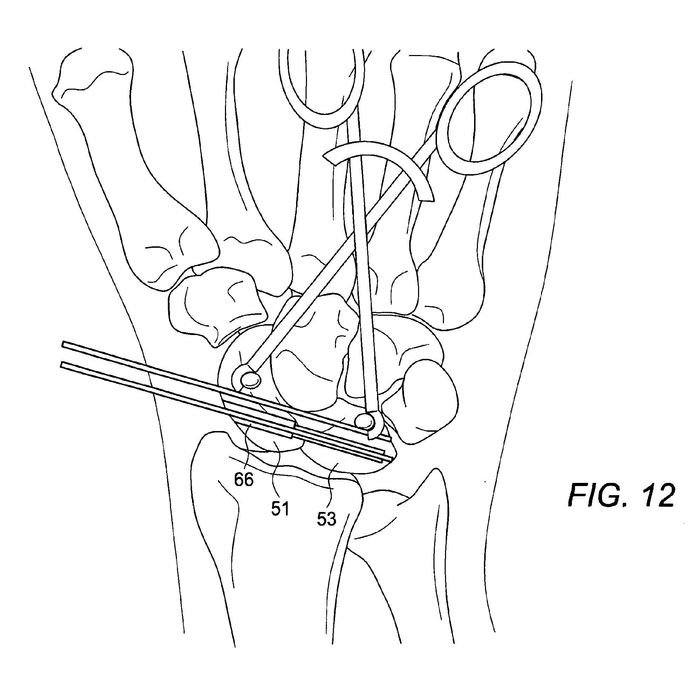

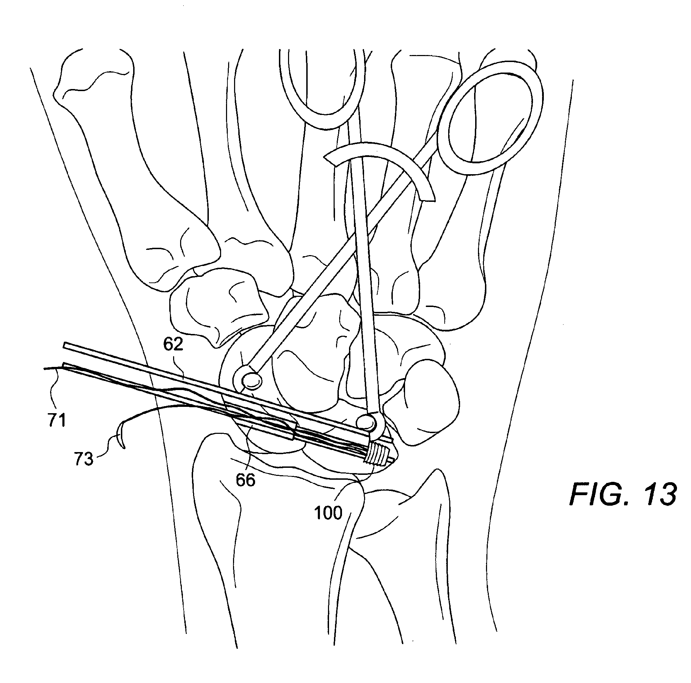

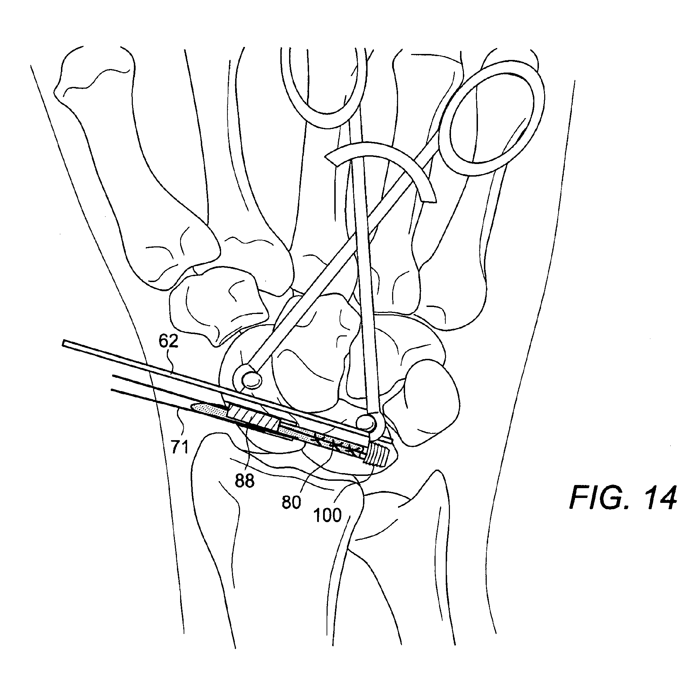

FIG. 12 shows removal of the stepped drill 65 and formation of hole 66 (through both the scaphoid 51 and the lunate 53). The implant 100 (3.5mm scapholunate threaded anchor 100 of FIGS. 1A-1F) preloaded with a suture strand 71 and a needle 73 is placed into the hole 66 in the ulnar portion of the lunate 53, as shown in FIG. 13. Strand 71 and needle 73 are employed in suturing/stitching tendon 80 (FIG. 14) which may then be looped through the eyelet 20 of anchor 100 with its free tails/strands extend along smooth sides 16, 17 of the anchor body. Free tails/strands of the tendon 80 are then pulled tight while reduction of the scapholunate interval is maintained.

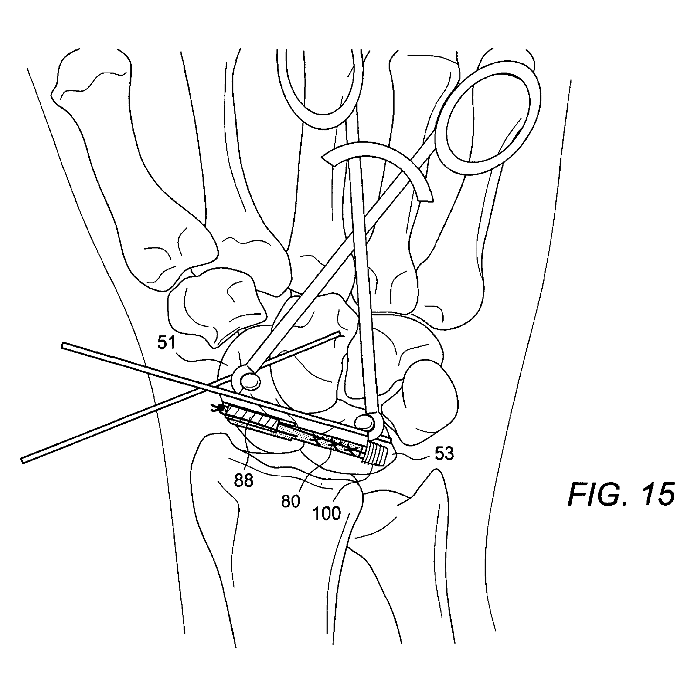

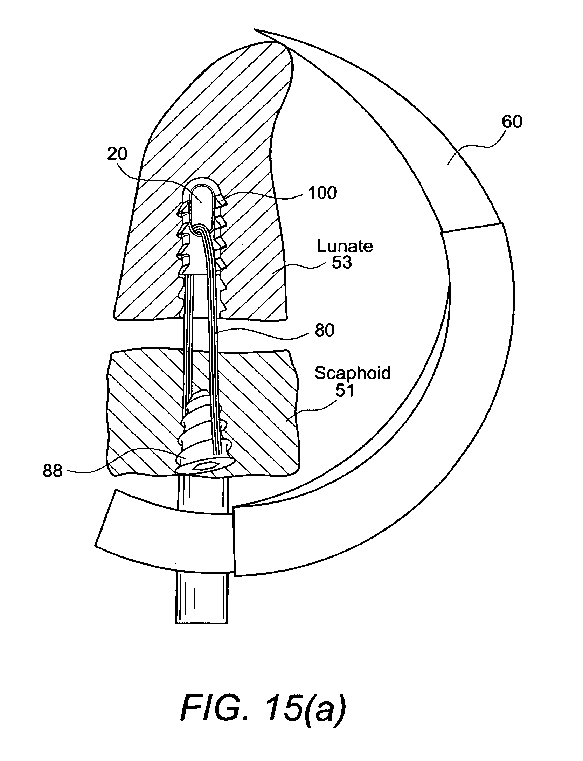

A fixation device 88 (FIGS. 14 and 15), for example, an interference screw 88 (a tenodesis screw 88) formed of PEEK, PLLA, or any biocompatible material including metals, for example, is inserted to complete fixation of the tendon 80 into the scaphoid 51. FIG. 15 shows the final repair with tendon 80 fixated within both the scaphoid 51 and the lunate 53 by the 3.5mm scapholunate threaded anchor 100 and by the tenodesis screw 88. FIG. 15(a) is another schematic illustration of the final scapholunate interosseous ligament repair of FIG. 15, showing threaded anchor 100 and the tenodesis screw 88 fixating the tendon 80.

FIGS. 16-22 illustrate subsequent steps of a second method of scapholunate interosseous ligament reconstruction (with an alternate graft docking with a suture loop). The method steps shown in FIGS. 16-22 are similar to those of FIGS. 5-15 but differ in that a suture loop is employed to attach the tendon graft 80 to the eyelet 20 of the 3.5mm scapholunate threaded anchor 100 of FIG. 1. As in the previously-described embodiment, the fixation of the tendon graft is achieved with the 3.5mm scapholunate threaded anchor 100 of FIGS. 1A-1F and a second fixation device (for example, an interference screw).

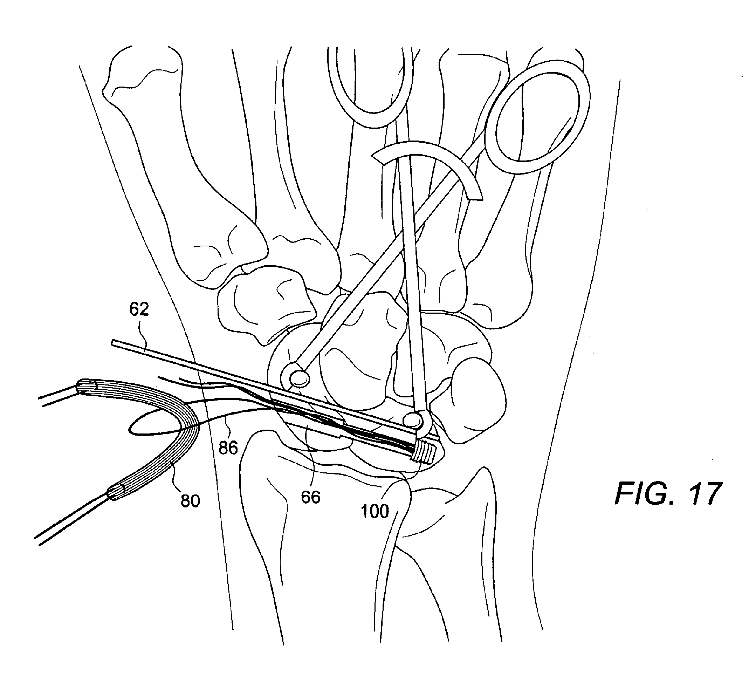

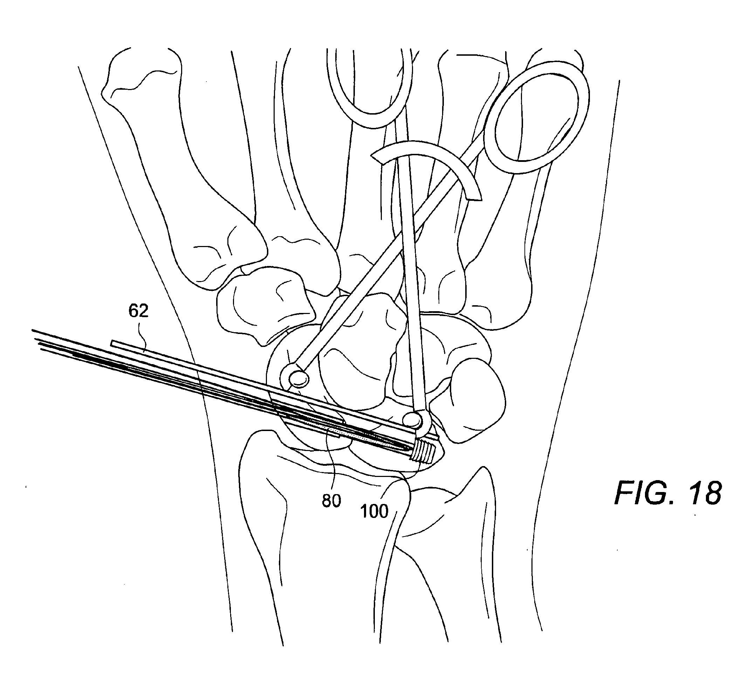

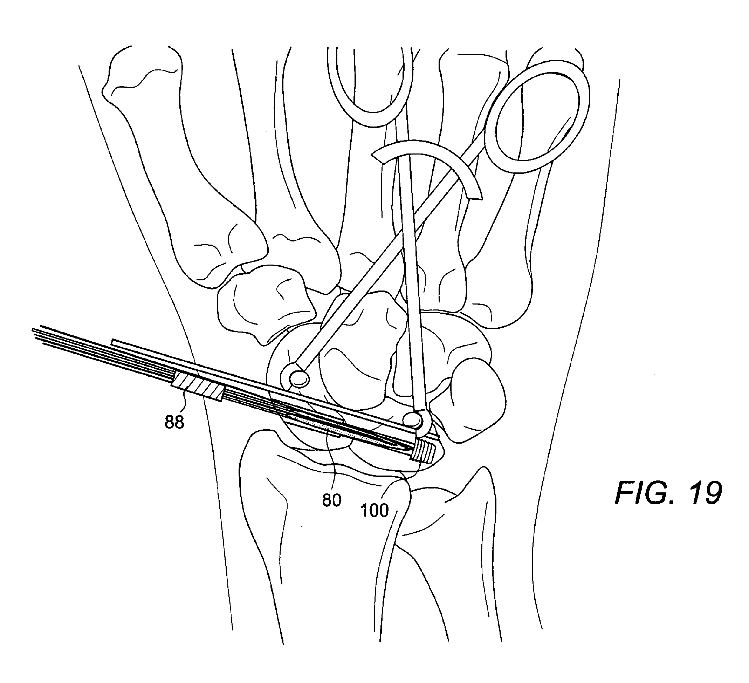

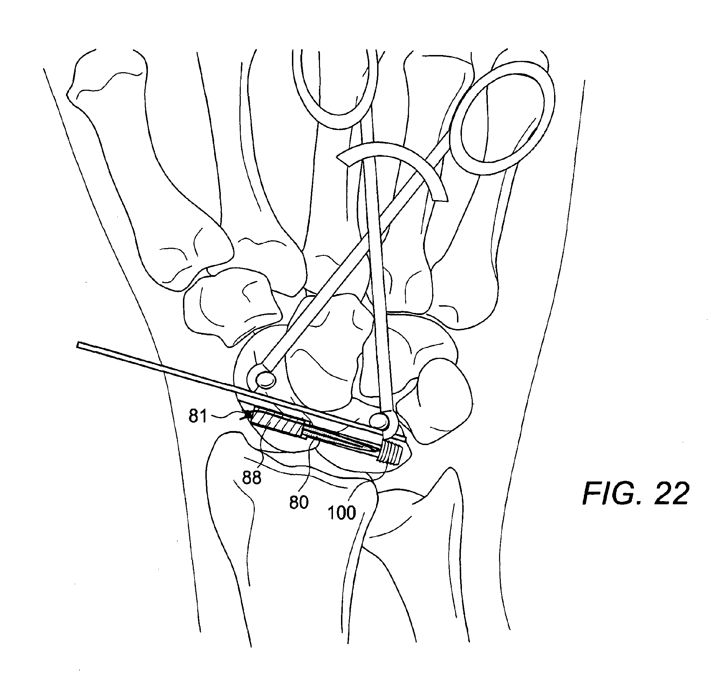

FIG. 16 corresponds to FIG. 12 but with the first K-wire 61 removed and with suture loop 86 attached to anchor 100 (in *lieu* of the suture strand 71 and needle 73). Graft 80 is passed through the suture loop 86 and looped over it, as shown in FIG. 17. Graft 80 and loop 86 are then pulled through hole 66, as shown in FIGS. 18 and 19, and then secured with a second fixation device 88 (for example, an interference screw or tenodesis screw 88), as shown in FIGS. 19-21. The final repair is shown in FIG. 22 with tendon 80 fixated within both the scaphoid 51 and the lunate 53 by the 3.5mm scapholunate threaded anchor 100, by the suture loop 86 and by the tenodesis screw 88 (with knot 81).

Although the exemplary methods above have been described with reference to a biological material (such as tendon graft 80) that is "drawn" into the tunnel by pulling the suture tails through the eyelet 20 of the 3.5mm scapholunate threaded anchor 100, the biological or synthetic material (tendon graft 80) may also be looped directly through the eyelet 20 (and folded over the eyelet 20) before it is deployed into the bony tunnel or socket, to achieve the final construct of FIG. 15(a). Optionally, and if desired, the folded over tendon graft 80 may be secured to the body 10 of the anchor 100 by additional suturing/stitching.

The devices of the present invention have applicability to any type of interosseous ligament reconstruction, the scapholunate interosseous ligament repairs detailed above being just two examples. In the disclosed methods, the graft is directly connected to, or through, the anchor/implant, The design of the anchor of the present invention employs an eyelet for the biological graft, while the prior art designs retain the graft only by interference fit. The design of the anchor of the present invention also allows the use of a biologic graft and fixation of such graft, while the prior art designs permit fixation of only synthetic materials. The methods detailed above are also considered single-approach surgical procedures, in that anchoring of multiple anchors and/or fixation devices is conducted in-line, and through a single portal, resulting in minimally invasive repairs.

Although the present invention has been described in relation to particular embodiments thereof, many other variations and modifications and other uses will become apparent to those skilled in the art. Therefore, the present invention is to be limited not by the specific disclosure herein, but only by the appended claims,

## Claims

1. An anchor (100, 200, 300) for interosseous tissue reconstruction and scapholunate, hallux valgus or syndesmotic repairs, comprising: a body (10) with a central axis (1,1), a proximal end (12), a distal end (14), an internal bore (15, 215, 315) provided at the proximal end (12); two opposing flat lateral sides (16, 17), and other two opposing lateral sides (18, 19) that are adjacent the two opposing flat lateral sides (16, 17) and are provided with external non-flat surfaces (21), and an eyelet (20) located at the distal end (14) of the body; **characterized in that**
the body has a length of about 3.5mm and an eyelet which is about half the length of the body;
so that tissue can be looped through and over the eyelet (20) of the anchor, and extending along the two opposing flat lateral sides (16, 17) of the anchor and in a direction about parallel to the central axis (11) of the body of the anchor, when the anchor is inserted into a socket or tunnel in a first bone and the tissue is secured into a second bone adjacent the first bone.

2. The anchor of claim 1, wherein the internal bore extends from the most proximal end (12) of the body and is in direct communication with the eyelet.

3. The anchor of claim 1 or claim 2, wherein the internal bore is threaded.

4. The anchor of any claim 1 to 3, wherein the two opposing flat lateral sides are provided with smooth, non-threaded and non-ribbed outer surfaces.

5. The anchor of any claim 1 to 3, wherein the external non-flat surfaces of the other two opposing lateral sides are provided with a plurality of partial threads or partial ribs.

6. The anchor of any claim 1 to 4 provided for a tissue which is a biological material or a synthetic material.

7. The anchor of claim 5 wherein the tissue is one of a graft, tendon or ligament.

8. The anchor of any claim 1 to 6 designed for scapholunate repairs.

9. The anchor of any claim 1 to 7, wherein the anchor is pre-loaded with a suture and a needle.

10. The anchor of any claim 1 to 8 wherein the anchor is provided with a continuous, uninterrupted flexible loop of a flexible strand attached to the eyelet.

## Patentansprüche

1. Anker (100, 200, 300) für intraossäre Geweberekonstruktion und Reparatur von Scapholunat, Hallux Valgus und Syndesmose, umfassend einen Körper (10) mit einer zentralen Achse (11), einem proximalen Ende (12), einem distalen Ende (14) einer am proximalen Ende (12) bereitgestellten inneren Bohrung (15, 215, 315); zwei entgegengesetzte flache laterale Seiten (16, 17) und zwei weitere entgegengesetzte laterale Seiten (18, 19), die sich neben den entgegengesetzten flachen lateralen Seiten (16, 17) befinden und mit äußeren, nicht flachen Oberflächen (21) ausgestattet sind, und eine Öse (20), die sich am distalen Ende (14) des Körpers befindet; **dadurch gekennzeichnet, dass**
der Körper eine Länge von etwa 3,5 mm hat und eine Öse, die etwa die halbe Länge des Körpers hat;
so dass Gewebe durch und über die Öse (20) des Ankers gewunden werden kann, und sich entlang den zwei entgegengesetzten flachen lateralen Seiten (16, 17) des Ankers erstrecken kann, in einer Richtung, etwa parallel zur zentralen Achse (11) des Körpers des Ankers, ist der Anker in eine Halterung oder einen Tunnel in einem ersten Knochen eingeführt, und ist das Gewebe in einem zweiten Knochen neben dem ersten Knochen befestigt.

2. Anker gemäß Anspruch 1, wobei die innere Bohrung sich vom proximalsten Ende (12) des Körpers aus erstreckt und in direkter Verbindung mit der Öse ist.

3. Anker gemäß Anspruch 1 oder Anspruch 2, wobei die innere Bohrung ein Gewinde aufweist.

4. Anker gemäß irgendeinem der Ansprüche 1 bis 3, wobei die entgegengesetzten flachen lateralen Seiten mit glatten äußeren Oberflächen ohne Gewinde und ohne Rippen ausgestattet sind.

5. Anker gemäß irgendeinem der Ansprüche 1 bis 3, wobei die äußeren nicht flachen Oberflächen der zwei weiteren entgegengesetzten lateralen Seiten mit einer Mehrzahl teilweisen Gewinden oder teilweisen Rippen ausgestattet sind.

6. Anker gemäß irgendeinem der Ansprüche 1 bis 4, bereitgestellt für ein Gewebe, das ein biologisches Material ist oder ein synthetisches Material.

7. Anker gemäß Anspruch 5, wobei das Gewebe eines ist aus einem Transplantat, einer Sehne oder einem Band.

8. Anker gemäß irgendeinem der Ansprüche 1 bis 6, ausgelegt für Reparaturen von Scapholunat.

9. Anker gemäß irgendeinem der Ansprüche 1 bis 7, wobei der Anker vorbeladen ist mit einer Naht und einer Nadel.

10. Anker gemäß irgendeinem der Ansprüche 1 bis 8, wobei der Anker ausgestattet ist mit einer durchgehenden, nicht unterbrochenen flexiblen Schlaufe eines flexiblen Garns, befestigt an der Öse.

## Revendications

1. Une ancre (100, 200, 300) pour la reconstruction de tissus interosseux et des réparations scapho-lunaires, de l'hallux valgus ou syndesmotiques, comprenant : un corps (10) avec un axe central (11), une extrémité proximale (12), une extrémité distale (14), un orifice interne (15, 215, 315) situé au niveau de l'extrémité proximale (12), deux côtés latéraux plats opposés (16, 17) et deux autres côtés latéraux opposés (18, 19) qui sont adjacents aux deux côtés latéraux plats opposés (16, 17) et sont munis de surfaces non planes externes (21), et un oeillet (20) situé au niveau de l'extrémité distale (14) du corps, **caractérisé en ce que**
le corps a une longueur d'environ 3,5 mm et un oeillet qui est environ la moitié de la longueur du corps,
de sorte qu'un tissu puisse être passé en boucle au travers de et par dessus l'oeillet (20) de l'ancre et s'étendre le long des deux côtés latéraux plats opposés (16, 17) de l'ancre et dans une direction approximativement parallèle à l'axe central (11) du corps de l'ancre, lorsque l'ancre est insérée dans une cavité ou un tunnel dans un premier os et le tissu est rattaché à un deuxième os adjacent au premier os.

2. L'ancre selon la revendication 1, où l'orifice interne s'étend de l'extrémité la plus proximale (12) du corps et est en communication directe avec l'oeillet.

3. L'ancre selon la revendication 1 ou 2, où l'orifice interne est fileté.

4. L'ancre selon l'une quelconque des revendications 1 à 3, où les deux côtés latéraux plats opposés sont munis de surfaces extérieures lisses, non filetées et non nervurées.

5. L'ancre selon l'une quelconque des revendications 1 à 3, où les surfaces non planes externes des deux autres côtés latéraux opposés sont munis d'une pluralité de filets partiels ou de nervures partielles.

6. L'ancre selon l'une quelconque des revendications 1 à 4 destinée à un tissu qui est un matériau biologique ou un matériau synthétique.

7. L'ancre selon la revendication 5 où le tissu est un tissu parmi un greffon, un tendon ou un ligament.

8. L'ancre selon l'une quelconque des revendications 1 à 6 conçue pour des réparations scapho-lunaires.

9. L'ancre selon l'une quelconque des revendications 1 à 7, où l'ancre est préchargée avec une suture et une aiguille.

10. L'ancre selon l'une quelconque des revendications 1 à 8 où l'ancre est munie d'une boucle flexible continue et non interrompue d'un fil flexible attaché à l'oeillet.
